# EUROPÄISCHE PATENTSCHRIFT

(11) **EP 3 777 564 B1**
(45) Veröffentlichungstag und Bekanntmachung des Hinweises auf die Patenterteilung: **23.03.2022**
(21) Anmeldenummer: 20173968.7
(22) Anmeldetag: 11.05.2020
(51) Int. Cl.: C09D 191/06, A47G 21/18, A47G 21/00, A61B 13/00, A61C 17/00, A23L 29/212, A23L 29/262, A23L 29/269, C08L 3/02, C08L 5/00, C08L 5/12, A23L 29/00, A23L 29/238

(54) **BIOLOGISCH ABBAUBARE HILFSMITTEL ZUM ESSEN ODER TRINKEN AUS PFLANZLICHER STÄRKE UND PFLANZLICHEM DICKUNGS- ODER GELIERMITTEL UND VERFAHREN ZUR HERSTELLUNG DERSELBEN**
BIODEGRADABLE AID FOR EATING OR DRINKING MADE FROM VEGETABLE STARCH AND VEGETABLE THICKENING OR GELLING AGENT AND METHOD FOR PRODUCING THE SAME
AUXILIAIRE BIODÉGRADABLE DESTINÉ À L'ALIMENTATION SOLIDE OU LIQUIDE À PARTIR D'AMIDON VÉGÉTAL ET D'AGENTS D'ÉPAISSISSEMENT OU DE GÉLIFICATION VÉGÉTAUX ET SON PROCÉDÉ DE FABRICATION

(30) Priorität: 13.08.2019 DE 102019212126
(43) Veröffentlichungstag der Anmeldung: 17.02.2021
(73) Patentinhaber: Hope Tree International GmbH, 83607 Holzkirchen (DE)
(72) Erfinder: Dinzinger, Lambert Dustin, 82538 Geretsried (DE)
(74) Vertreter: Grund, Martin

(56) Entgegenhaltungen:
- WO-A1-2019/046789
- WO-A2-2007/062265

## Beschreibung

### Gebiet der Erfindung

Die vorliegende Erfindung liegt auf dem Gebiet von biologisch abbaubaren Hilfsmitteln zum Essen oder zum Trinken umfassend Trinkhalme / Strohhalme und Besteck und Verfahren zu deren Herstellung. Unter Verwendung von pflanzlicher Stärke und pflanzlichem Dickungs- oder Geliermittel können recycelbare und biologisch abbaubare Hilfsmittel zum Essen oder zum Trinken hergestellt werden. Die Zugabe von Zellstoff, Holzstoff und/oder Wachs, insbesondere Carnaubawachs, kann den Hilfsmitteln zum Essen oder zum Trinken weitere vorteilhafte Eigenschaften verleihen. Hierbei können Hilfsmittel zum Essen oder zum Trinken in halb-transparenter oder opaker Ausführungsform hergestellt werden. Optional kann durch Zugabe von Lebensmittelfarbe die Farbe modifiziert werden.

### Hintergrund der Erfindung

Allein in Deutschland werden täglich tausende Trinkhalme / Strohhalme und Einweg-Besteck verbraucht. Herkömmliche Einweg-Trinkhalme und Einweg-Bestecke sind weder recycle- noch kompostierbar, sie können nur verbrannt oder unter enormem Kosten- und Energieaufwand verwertet werden und schaden so dem Erhalt unseres Ökosystems. Infolgedessen ist der Bedarf an einer umweltfreundlichen, biologisch abbaubaren und kompostierbaren Alternative zu herkömmlichen Trinkhalmen und Besteck aber auch anderen Hilfsmitteln zum Essen oder zum Trinken wie kompostierbare Lollisticks enorm groß.

Eine solche Alternative bieten die Hilfsmittel zum Essen oder zum Trinken der vorliegenden Erfindung, sowie die Verfahren zu deren Herstellung. Während herkömmliche Hilfsmittel zum Essen oder zum Trinken wie Trinkhalme und Besteck aus Plastik bestehen, bestehen Hilfsmittel zum Essen oder zum Trinken der vorliegenden Erfindung aus rein pflanzlichen Materialien, wie pflanzlicher Stärke, pflanzlichem Dickungs- oder Geliermittel, Zellstoff, Holzstoff und Carnaubawachs. Die z.B. Trinkhalme und das Besteck sind somit nicht nur leicht zu recyceln bzw. zu kompostieren, sie werden auch aus nachwachsenden Rohstoffen produziert, die eine positive CO₂-Bilanz aufweisen. Daher bilden die Hilfsmittel zum Essen oder zum Trinken der vorliegenden Erfindung eine umweltfreundliche Alternative zu gebräuchlichen Lösungen wie Einweg-Trinkhalmen aus Plastik.

In WO 2019/046789 werden essbare und/oder biologisch abbaubare Gefäße wie Trinkhalme und Becher beschrieben. Diese Gefäße umfassen ein Hydrokolloid wie Agar oder Alginat und Flüssigkeit.

WO 2007/062265 offenbart Materialien für biologisch abbaubare Gefäße.

### Zusammenfassung der Erfindung

Die Erfindung wird durch die Ansprüche definiert.

Entsprechend bezieht sich die vorliegende Erfindung auf Hilfsmittel zum Essen und zum Trinken und Verfahren zu deren Herstellung.

Die vorliegende Erfindung bezieht sich auf Hilfsmittel zum Essen oder zum Trinken umfassend
40-50% pflanzliche Stärke,
35-45% Guarkernmehl und
1-5% Xanthan.

Die Hilfsmittel zum Essen oder zum Trinken gemäß der vorliegenden Erfindung können ferner eine äußere Wachsschicht umfassend Carnaubawachs, Rapswachs, Paraffin, PHA oder Mischungen davon umfassen.

In manchen Ausführungsformen ist das pflanzliche Dickungs- oder Geliermittel eine Mischung aus Guarkernmehl und Xanthan.

Das Hilfsmittel zum Essen oder zum Trinken gemäß der vorliegenden Erfindung kann ein Trinkhalm, ein Besteck oder ein Lollistiel sein. Das Hilfsmittel zum Trinken der vorliegenden Erfindung ist bevorzugt ein Trinkhalm. Das Hilfsmittel zum Essen der vorliegenden Erfindung ist bevorzugt ein Lollistiel.

Die Hilfsmittel zum Essen oder zum Trinken gemäß der vorliegenden Erfindung sind kompostierbar und/oder vollständig biologisch abbaubar.

Die vorliegende Erfindung betrifft ferner ein Verfahren zur Herstellung von Hilfsmitteln zum Essen oder zum Trinken.

Die vorliegende Erfindung bezieht sich auf ein Verfahren zur Herstellung von Hilfsmitteln zum Essen oder Trinken, wobei das Verfahren die folgenden Schritte umfasst:
a. Herstellen einer Mischung, die pflanzliche Stärke und pflanzliches Dickungs- oder Geliermittel umfasst, umfassend die Schritte
   a.1 Zusammenfügen der festen Bestandteile der Mischung umfassend pflanzliche Stärke und pflanzliches Dickungs- und Geliermittel und Mischen dieser Bestandteile,
   a.2 Zugabe von flüssigen Bestandteilen umfassend Wasser zur Mischung aus Schritt a.1 in einer Menge von 30-60% des Nettogewichts der Mischung aus a.1 und Mischen dieser Bestandteile,
b. Formen der Mischung aus Schritt a. zu einem Hilfsmittel zum Essen oder Trinken, wobei die Mischung aus Schritt a vor dem Formen mittels einer Plastifizierschnecke verdichtet wird,
c. Aushärten des in Schritt b. geformten Hilfsmittels zum Essen oder Trinken,
d. optional, Beschichten des in Schritt c. ausgehärteten Hilfsmittels zum Essen oder Trinken mit einer

Wachsemulsion umfassend Carnaubawachs, Rapswachs, Paraffin, PHA oder Mischungen davon. In manchen Ausführungsformen ist die pflanzliche Stärke in der Mischung in Schritt a.1 Weizenstärke, Kartoffelstärke, Maisstärke, Tapiokastärke oder eine Mischung davon.

In manchen Ausführungsformen umfassen die flüssigen Bestandteile aus Schritt a.2 ferner Öl, bevorzugt Nussöl oder Sonnenblumenöl, Glycerin und/oder eine Wachsemulsion.

In einer bevorzugten Ausführungsform umfasst das Verfahren zur Herstellung von Hilfsmitteln zum Essen oder Trinken die folgenden Schritte:
a. Herstellen einer Mischung, die pflanzliche Stärke und pflanzliches Dickungs- oder Geliermittel umfasst, umfassend die Schritte
   a.1 Zusammenfügen der festen Bestandteile der Mischung umfassend
      50% Weizenstärke
      45% Guarkernmehl
      5% Xanthan
      und Mischen dieser Bestandteile,
   a.2 Zugabe von flüssigen Bestandteilen der Mischung zur Mischung aus Schritt a.1 umfassend bezogen auf das Nettogewicht der Mischung aus Schritt a.1
      2% Carnaubawachsemulsion
      4% Sonnenblumenöl
      5% Glycerin und
      40% Wasser
      und Mischen dieser Bestandteile
b. Formen der Mischung aus Schritt a. zu einem Hilfsmittel zum Essen oder Trinken, wobei die Mischung aus Schritt a vor dem Formen mittels einer Plastifizierschnecke verdichtet wird,
c. Aushärten des in Schritt b. geformten Hilfsmittels zum Essen oder Trinken,
d. optional, Beschichten des in Schritt c. ausgehärteten Hilfsmittels zum Essen oder Trinken mit einer Wachsemulsion umfassend Carnaubawachs, Rapswachs, Paraffin, PHA oder Mischungen davon.

Die Wachsemulsion im optionalen Schritt d. kann dabei entweder aus 32,5% Carnaubawachs, 17,5% Paraffin und 50% Wasser oder aus 50% Carnaubawachs und 50% Wasser besteht.

### Vorteile der Erfindung

Durch die Verwendung der pflanzlichen Stärke und pflanzlichen Dickungs- und Geliermittel sind die Hilfsmittel zum Essen oder zum Trinken der vorliegenden Erfindung im Gegensatz zu herkömmlichen Einweg-Lösungen wie Plastiktrinkhalme oder Plastikbesteck recycelbar, vollständig biologisch abbaubar und auch kompostierbar. So wurde die Kompostierdauer eines erfindungsgemäßen Trinkhalms, welcher hergestellt wurde aus einer Mischung umfassend 50% Stärke, 45% Guarkernmehl 5% Xanthan und bezogen auf das Nettogewicht dieser Bestandteile 2% Carnaubawachsemulsion, 40% Wasser und 4% Öl von einer zuständigen Behörde (DEKRA) als Lebensmittelecht getestet. Ferner wurde dieser Trinkhalm auf seine Kompostierbarkeit getestet. Die Testung ergab eine Kompostierdauer des Trinkhalms von 22 Tagen. Dieser Wert liegt weit unter dem Wert alternativer Trinkhalme.

In manchen Ausführungsformen umfassen die Hilfsmittel zum Essen oder zum Trinken der Erfindung Zellstoff. Dies erhöht die mechanische Stabilität der Hilfsmittel zum Essen oder zum Trinken und kann so das Abknicken oder Zerreisen dieser verhindern. Die Hilfsmittel und die Bedarfsartikel der vorliegenden Erfindung erhalten bis zu 15% Zellstoff. Ein höherer Zellstoff-Gehalt würde die Hilfsmittel oder Bedarfsmittel zu spröde machen. Besonders gute mechanische Eigenschaften ergeben sich bei einem Zellstoff-Gehalt von 2-10%.

In manchen Ausführungsformen umfassen die Hilfsmittel zum Essen oder zum Trinken der Erfindung Holzstoff. Wie Zellstoff erhöht auch Holzstoff die mechanische Stabilität. Im Vergleich zu Holzstoff hat Zellstoff den Vorteil, dass die Hilfsmittel oder Bedarfsartikel eine höhere Transparenz aufweisen.

In manchen Ausführungsformen umfassen die Hilfsmittel zum Essen oder zum Trinken der Erfindung Wachs, bevorzugt Carnaubawachs oder Sojawachs. Wachs erhöht die Resistenz der Hilfsmittel zum Essen oder zum Trinken gegenüber Wasser und Öl. Das höchst widerstandfähige Carnaubawachs kann die Stabilität während der Benutzung in einer Flüssigkeit um mindestens 10%, mindestens 20%, mindestens 30% oder sogar um mehr als 30% steigern. Auch die die Verwendung von Sojawachs zeigt diesen Effekt.

In manchen Ausführungsformen umfassen die Hilfsmittel zum Essen oder zum Trinken der Erfindung Öl, bevorzugt Nussöl. Dies verbessert die Fließeigenschaften der Rohmasse für die Hilfsmittel zum Essen oder zum Trinken während der Herstellung.

Der Anteil an pflanzlichen Dickungs- und Geliermittel beeinflusst ebenso die Transparenz des Materials. Je höher der Anteil der pflanzlichen Dickungs- oder Geliermittel, desto höher wird die Transparenz.

In manchen Ausführungsformen umfassen die Hilfsmittel zum Essen oder zum Trinken der Erfindung Magnesiumstearat und/oder Calciumstearat und/oder Zinkstearat. Magnesium- und Calciumstearat werden hierbei als Schmiermittel, Antihaftmittel und Geliermittel für eine verbesserte Fließeigenschaft der Rohmasse während der Herstellung verwendet. Die Zugabe von Zinkstearat verbessert die Aufnahme der Rohmasse in beim Herstellungsverfahren verwendeten Vorrichtungen, z.B. das Einziehen der Rohmasse mittels einer Plastifizierschnecke. In manchen Ausführungsformen wird im Herstellungsverfahren ein Teil des zugegebenen Wassers durch Glycerin E422 ersetzt, um die Beweglichkeit bzw. die Biegeeigenschaft der erfindungsgemäßen Hilfsmittel zum Essen oder zum Trinken zu erhöhen.

### Ausführliche Beschreibung

### Definitionen

**"Hilfsmittel zum Essen oder zum Trinken"** umfassen Trinkhalme, Besteck, Lollisticks / Lollistiele, Sushi-Stäbchen und Eisstiele.

**"Trinkhalm"** und **"Strohhalm"** werden in der vorliegenden Anmeldung synonym verwendet.

**"Besteck"** ist ein Sammelbegriff für verschiedene Werkzeuge, mit denen Lebensmittel serviert, zerkleinert und gegessen werden. Besteck umfasst dementsprechend Messer, Gabeln, Pommesgabeln, Esslöffel, Teelöffel, Kuchengabeln, Eislöffel, sowie Abwandlungen hiervon.

**"Pflanzliche Stärke"** bezieht sich auf jegliche aus Pflanzenmaterial gewonnene Stärke. Die Stärke kann dabei etwa aus Wurzeln, Rüben, Knollen, Rhizomen, Sprossachsen, Blättern, Früchten oder Samen gewonnen werden. Beispielhaftes pflanzliche Stärken sind Weizenstärke, Kartoffelstärke, Maisstärke oder Tapiokastärke; Stärke aus Maniok (*Manihot esculenta*), Knollenbohne (*Pachyrhizus tuberosus),* Batate (*Ipomoea batatas*), Yamswurzel (*Dioscorea spec.*)*,* Knollen-Platterbse (*Lathyrus tuberosus),* Arakacha (*Arracacia xanthorrhiza*), Knolligem Sauerklee (*Oxalis tuberosa*), Knolliger Kapuzinerkresse (*Tropaeolum tuberosum*)*,* Ulluco (*Ullucus tuberosus*)*,* Ostindischer Pfeilwurz (*Tacca leontopetaloides*), Pfeilwurz (*Maranta spec.),* Achira (*Canna indica*), Taro (*Colocasia esculenta*), Tannia (*Xanthosoma sagittifolium*), Weißer Seerose (*Nymphaea alba*), Gelber Teichrose (*Nuphar lutea*) oder Chayote (*Sechium edule).*

**"Faserstoff"** bezeichnet aus Fasern gewonnenes Material, das für die Herstellung von Papier und Pappe verwendet wird. Faserstoffe bestehen zum einem Großteil aus Cellulose. Faserstoffe sind beispielsweise Zellstoff und Holzstoff.

**"Zellstoff"** bezeichnet die beim chemischen Aufschluss von Pflanzen, v.a. Holz, entstehende faserige Masse. Sie besteht zu einem Großteil aus Cellulose. Für die Trinkhalme der vorliegenden Erfindung wird bevorzugt Zellstoff mit kurzen Fasern verwendet. Eine besonders bevorzugte Faserlänge ist 0,8 mm bis 1,1 mm. Der Zellstoff der vorliegenden Erfindung kann von vielerlei Pflanzen stammen, etwa von Nadelbäumen, Laubbäumen oder Bambus. In besonders bevorzugten Ausführungsformen ist der Zellstoff zumindest teilweise aus Baobab-Pflanzenmaterial hergestellt. In einer anderen besonders bevorzugten Ausführungsform ist der Zellstoff zumindest teilweise aus Bambus-Pflanzenmaterial hergestellt.

**"Holzstoff"** bezeichnet die bei mechanischem Aufschluss von Pflanzen, v.a. Holz, entstehende faserige Masse. Holzstoff enthält, anders als Zellstoff für höherwertige Papiere, große Anteile an Lignin. Für die Hilfsmittel zum Essen oder zum Trinken der vorliegenden Erfindung wird bevorzugt Holzstoff mit kurzen Fasern verwendet. Eine besonders bevorzugte Faserlänge ist 0,8 mm bis 1,1 mm. In manchen Ausführungsformen der vorliegenden Erfindung kommt eine Kombination aus Holzstoff und Zellstoff zum Einsatz.

**"Pflanzliche Dickungsmittel"** und **"pflanzliche Geliermittel"** sind Dickungs- und Geliermittel aus pflanzlichen oder bakteriellen Ressourcen. Bevorzugt sind Dickungs- und Geliermittel aus pflanzlichen Ressourcen. Sie bewirken die Gelierung von Flüssigkeit. Beispielhafte pflanzliche Dickungs- oder Geliermittel sind Agar-Agar, Pektin, Carrageen, Alginate, Johannisbrotkernmehl, Guarkernmehl, Sago, Xanthan, Gummi Arabicum, Reismehl, Hartweizenmehl oder Hartweizengrieß.

**"Guarkernmehl"** (als Lebensmittelzusatzstoff E412) ist ein pflanzliches Dickungs- oder Geliermittel. Es wird aus gemahlenen Samen der Guarpflanze gewonnen. Besonders hervorzuheben ist, dass Guarkernmehl die Wirkung anderer pflanzlicher Verdickungs- oder Geliermittel erheblich verstärkt und daher gerne mit anderen pflanzlichen Verdickungs- oder Geliermitteln eingesetzt wird.

**"Xanthan"** (als Lebensmittelzusatzstoff E415, auch als "xanthan gum" bezeichnet), ist ein bakterielles Dickungs- und Geliermittel. Es wird durch Bakterien der Gattung *Xanthomonas* aus zuckerhaltigen Substraten hergestellt. Bevorzugt stammt das Xanthan der vorliegenden Erfindung von Bakterien der Spezies *Xanthomonas campestris.*

**"Agar-Agar"** bezeichnet ein aus Algen gewonnenes Galactose-Polymer. Agar-Agar wird aus den Zellwänden von Algen, insbesondere von Rotalgen gewonnen.

**"Pektin"** (als Lebensmittelzusatzstoff E440a oder E440b) bezeichnet pflanzliche Polysaccharide, die im Wesentlichen α-1-4-glycosidisch verknüpfte Galacturonsäuren umfassen. Sie können zum Beispiel aus Schalen von Äpfeln, Zitronen und anderen Früchten gewonnen werden. Pektin ist ein pflanzliches Dickungs- oder Geliermittel und sorgt so dafür, dass Flüssigkeiten gelieren.

**"Carrageen"** (als Lebensmittelzusatzstoff E407) ist ein pflanzliches Dickungs- oder Geliermittel, das aus verschiedenen Rotalgenarten gewonnen wird.

**"Alginat"** ist ein pflanzliches Dickungs- oder Geliermittel und besteht aus Salzen der Alginsäure. Es kann aus getrockneten und gemahlenen Braunalgen extrahiert werden. Je nach Salz ist es unter den E-Nummern E401, E402, E403, E404 und E405 bekannt.

**"Johannisbrotkernmehl"** (als Lebensmittelzusatzstoff E410) ist ein pflanzliches Dickungs- oder Geliermittel, das aus Johannisbrotbaumsamen durch mahlen gewonnen wird. Das erhaltene Mehl ist weiß und geschmacksneutral.

**"Sago"** ist ein pflanzliches Dickungs- oder Geliermittel. Sago wird aus dem stärkereichen Mark verschiedener Pflanzenarten wie der Sagopalme, Maniok, oder Kartoffeln gewonnen. Häufig wird es als Granulat in Form kleiner Kügelchen angeboten. Sago quillt in heißer Flüssigkeit etwa um das Dreifache auf und wirkt beim Abkühlen stark bindend. Damit die Flüssigkeit nicht breiig wird, wird Sago nur so lange gekocht oder eingeweicht, bis die Kügelchen weich sind, aber noch ihre Form behalten.

**"Gummi arabicum"** (als Lebensmittelzusatzstoff E414) ist ein pflanzliches Dickungs- und Geliermittel, das aus dem harzigen Pflanzensaft von in Afrika beheimateten Akazienarten, wie beispielsweise *Acacia senegal*, gewonnen wird. Gummi arabicum kann sowohl in Pulver- als auch in Gummiform vorliegen.

**"Reismehl"** ist ein pflanzliches Dickungs- und Geliermittel, das durch die Entspelzung von Reiskörnern und anschließendes feines Mahlen entsteht. Je nach Verwendung von poliertem oder unpoliertem (braunen) Reiskörnern, erhält man weißes oder braunes Reismehl.

**"Hartweizenmehl"** ist ein pflanzliches Dickungs- und Geliermittel, das aus dem Hartweizen (*Triticum durum*) gewonnen wird, der auch unter Durum, Durumweizen oder Glasweizen bekannt ist. Die Hartweizenkörner werden geschält und anschließend mehrmals gemahlen, sodass Hartweizenmehl entsteht.

**"Hartweizengrieß"** ist ein pflanzliches Verdickungsmittel, das aus dem Hartweizen (*Triticum durum*) gewonnen wird, der auch unter Durum, Durumweizen oder Glasweizen bekannt ist. Wie bei der Hartweizenmehl-Herstellung werden die Hartweizenkörner geschält und anschließend gemahlen. Allerdings werden die Hartweißenkörner weniger häufig gemahlen als bei der Herstellung von Hartweizenmehl, so dass Hartweißengrieß eine gröbere Partikelstruktur aufweist.

**"Wachs"** bezeichnet jegliches in der Natur vorkommendes Wachs, wie etwa Carnaubawachs, Bienenwachs oder Sojawachs. Bevorzugt ist das Wachs in der vorliegenden Erfindung Carnaubawachs. **"Carnaubawachs"** wird aus dem Blatt der Carnaubapalme (*Copernicia prunifera*) gewonnen. Die Blätter werden geerntet, durch Trocknen und mechanisches Einwirkung wird das Wachs abgetrennt. Das Wachs kommt in diversen Industrien zum Einsatz, wie in der Lebensmittel-, Kosmetik- und Pharma-Industrie. Unbehandeltes Carnaubawachs hat eine helle gelbliche, grünliche bis dunkelgraue Farbe, es ist von Luftbläschen durchsetzt, hart, spröde und wasserunlöslich. Carnaubawachs ist das härteste natürliche Wachs mit dem höchsten Schmelzpunkt von über 80 °C. Außerdem ist es verzehrbar, liegt also natürlich in Lebensmittelqualität vor und hat einen milden Geschmack. Dies ist bei Trinkhalmen und Besteck, das Carnaubawachs umfasst von Vorteil, da die Trinkhalme und Besteck so keinen Eigengeschmack aufweisen und auf natürliche Art und Weise ein Produkt in Lebensmittelqualität darstellen. Außerdem gestaltet die Zugabe von Carnaubawachs zu den anderen Stoffen wie pflanzliche Stärke, Agar-Agar und Zellstoff den Verarbeitungsprozess der Stoffe leichtgängiger, während die Festigkeit, Härte und Resistenz der zu erhaltenden Trinkhalme und Bestecke erhalten bleibt. In der vorliegenden Erfindung kommt Carnaubawachs bevorzugt in Pulverform zur Anwendung. **"Sojawachs"** wird aus reifen Sojabohnen (*Glycine max*) gewonnen. Die Sojabohnen werden geerntet und zunächst wird Sojaöl gewonnen. Dieses wird hydriert, unter Druck von ca. 200 bar und Temperaturen von etwa 140°C - 225 °C in der Anwesenheit eines metallischen Katalysators erhält man Sojawachs. Das Wachs kommt in diversen Industrien zum Einsatz, wie in der Kosmetik- und Kerzen-Industrie. Unbehandeltes Sojawachs hat eine sehr helle, cremeweiße, manchmal gelbliche Farbe, ist hart, spröde und wasserunlöslich. Sojawachs hat einen Schmelzpunkt von etwa 50 °C. Außerdem ist es verzehrbar und hat einen milden Geschmack. Dies ist bei Trinkhalmen und Besteck von Vorteil, da die Trinkhalme und Besteck so keinen Eigengeschmack aufweisen und ohne weiteren Aufarbeitungsschritt ein Produkt in Lebensmittelqualität darstellen. Außerdem gestaltet die Zugabe von Sojawachs zu den anderen Stoffen wie pflanzliche Stärke, Agar-Agar und Zellstoff den Verarbeitungsprozess der Stoffe leichtgängiger, während die Festigkeit, Härte und Resistenz der zu erhaltenden Trinkhalme und Bestecke erhalten bleiben. In der vorliegenden Erfindung kommt Sojawachs bevorzugt in Pulverform zur Anwendung.

**"Öl"** bezeichnet jegliches pflanzliche, mineralische oder tierische Öl. Bevorzugt ist das Öl in der vorliegenden Erfindung ein pflanzliches Öl. Besonders bevorzugt sind Nussöl und Sonnenblumenöl.

**"Kompostierbar"** bezeichnet die Eigenschaft eines Materials, nach 6 Monaten unter definierten aeroben Bedingungen zu 90% abgebaut zu werden. Die Kompostierbarkeit eines Materials kann nach DIN EN 13432, Version 2000-12, bestimmt werden.

**"Biologisch abbaubar"** bezeichnet die Charakteristik eines Prozesses, einen organischen Stoff biologisch, das heißt durch Lebewesen oder ihre Enzyme, vollständig abzubauen. Dabei findet der Abbau des Stoffes unter aeroben Bedingungen in höchstens 10 Jahren, bevorzugt in 5 Jahren, noch bevorzugter in 1 Jahr statt.

**"Recycelbar"** heißt, dass das Material nach seiner Verwendung (etwa als Trinkhalm) durch einen Aufarbeitungsprozess (Recycling) als Material zur Herstellung eines neuen, nicht zur Verbrennung gedachten Produkts verwendet werden kann. Dies steht im Gegensatz zu Materialien, die nach ihrer Verwendung entweder durch Verbrennung verwertet oder dauerhaft deponiert werden müssen. Recycelbare Materialien sind etwa Zellstoff, Papier, Pappe und Cellulosehydrat.

**"Magnesiumstearat"** Magnesiumstearat ist das Magnesiumsalz der Stearinsäure und gehört zu den Kalkseifen. Es wird aus Fetten und Ölen unter Spaltung ihrer Glyceride mittels Magnesium, Seifen und Glycerin gewonnen. Magnesiumstearat wird zum Beispiel in der pharmazeutischen Industrie als Hilfsmittel zur Tabletten- oder Granulatherstellung verwendet. Magnesiumstearat wird auch in einigen Süßigkeiten verwendet. Magnesiumstearat kann sowohl aus Fetten tierischer als auch pflanzlicher Herkunft hergestellt werden. Oft wird Soja-, Raps- oder Maiskeimöl verwendet. Die Substanz ist auch nützlich, weil sie schmierende Eigenschaften aufweist, die verhindern, dass Inhaltsstoffe während der Komprimierung von chemischen Pulvern zu festen Tabletten an den Herstellungsgeräten haften bleiben. Magnesiumstearat ist das am häufigsten verwendete Gleitmittel für Tabletten. Bei der Herstellung gepresster Bonbons wirkt Magnesiumstearat als Trennmittel und wird zum Binden von Zucker in Hartbonbons wie Minzen verwendet. Magnesiumstearat wird allgemein als Schmiermittel in einer Konzentration zwischen 0,25% und 5,0% in der Herstellung von Tabletten, Kapseln und anderen oralen Darreichungsformen verwendet. Aufgrund seiner langjährigen Verwendung als Hilfsstoff in der Pharma-, Lebensmittel- und Kosmetikindustrie ist die Sicherheit von Magnesiumstearat gut dokumentiert.

**"Calciumstearat"** Calciumstearat wird zur Herstellung sogenannter non-tox Stabilisatoren von Kunststoffen, bevorzugt in Verbindung mit Zinkstearat, aber auch Bariumstearat oder Magnesiumstearat verwendet. Weiterhin dient es als Gleitmittel in pharmazeutischen Produkten und als Schmierstoff (Staufferfett) in der Papier- und Metall-verarbeitenden Industrie, als Hydrophobierungsmittel für Baustoffe sowie in der Sandaufbereitung. Technisches Calciumstearat wird durch Reaktion von Calciumchlorid mit dem Natriumsalz der Stearinsäure (meist verunreinigt mit dem Natriumsalz der Palmitinsäure) und anschließendes Auswaschen von Natriumchlorid gewonnen. Es ist ein Imprägniermittel für Textilien. In Kunststoffen kann es als Säurefänger oder Neutralisator in Konzentrationen von bis zu 1000 ppm, als Schmiermittel und als Trennmittel wirken. Es kann in plastischen Farbmittelkonzentraten verwendet werden, um die Pigmentbenetzung zu verbessern. In Hart-PVC kann es die Verschmelzung beschleunigen, den Fluss verbessern und das Anschwellen des Stempels verringern. Zu den Anwendungen in der Körperpflege- und Pharmaindustrie gehören Tablettenformtrennmittel, Antihaftmittel und Geliermittel. Calciumstearat ist Bestandteil einiger Arten von Entschäumern. Außerdem ist Calciumstearat ein Antibackmittel, welches die feie Beweglichkeit von Feststoffen ermöglicht und so das Zusammenbacken pulverförmiger Bestandteile verhindert. Bei der Papierherstellung wird Calciumstearat als Schmiermittel verwendet, um einen guten Glanz zu erzielen, und um Staubbildung und Faltenrisse bei der Papier- und Kartonherstellung zu verhindern. Eine Zugabe von etwa 0,1 bis 10% ist möglich.

**"Zinkstearat"** ist ein weißes Pulver mit einem Schmelzpunkt von 130°C, einem Flammpunkt von 277°C und einer Selbstentzündungstemperatur von 420°C. Die Molekülmasse beträgt 632,3 g/mol. Zinkstearat ist nicht wasserlöslich. Zinkstearat wird als Stabilisator für Emulsionen verwendet. In der Kartenmagie verwendet man Zinkstearat als Kartenpuder, welches die Gleitfähigkeit von Spielkarten verbessert. Zinkstearat wird auch als Schmierstoff bei der Kunststoffverarbeitung verwendet. Es verhindert ein Festsitzen von Polyamidteilen untereinander und ist ein Hilfsmittel bei Plastifizierproblemen. Wenn die Plastifizierschnecke das Material nicht richtig einzieht, kann dies durch Zugabe von ca. 0,2% Zinkstearat verbessert werden, besonders bei Polyamid 6.0. Wenn die Plastifizierschnecke das Material nicht richtig einzieht, kann dies durch Zugabe von ca. 0,2 % Zinkstearat verbessert werden.

**"Glycerin"** Glycerin ist der Trivialname und die gebräuchliche Bezeichnung von Propan-1,2,3-triol und ist ein Fettstoff der in der vorliegenden Erfindung als Schmiermittel und Feuchthaltemittel eingesetzt wird. Bevorzugt ist E 422. E 422 wird überwiegend aus pflanzlichen Fetten und Ölen gewonnen. Die Herstellung aus synthetischen oder tierischen Stoffen ist ebenfalls möglich. Bevorzugt ist "vegetarisches Glycerin" oder "veganes Glycerin", welches durch die Umesterung von pflanzlichen Ölen hergestellt wird. Als Lebensmittelzusatzstoff ist E 422 für alle Nahrungsmittel allgemein zugelassen und es existiert auch keine Höchstmengenbeschränkung für die Verwendung von Glycerin. In der vorliegenden Erfindung erhöht die Zugabe von Glycerin die Beweglichkeit des Materials, was vor allem bei den erfindungsgemäßen Trinkhalmen nützlich sein kann.

### Ausführungsformen der Erfindung

Die hierin beschriebenen Hilfsmittel zum Essen oder zum Trinken können unterschiedliche Zusammensetzungen haben. Immer umfassen sie pflanzliche Stärke und die pflanzlichen Dickungs-oder Geliermittel Guarnkernmehl und Xanthan. Zusätzlich sind folgende pflanzliche Dickungs- oder Geliermittel möglich Agar-Agar, Pektin, Carrageen, Alginat, Johannisbrotkernmehl, Sago, Gummi arabicum, Reismehl, Hartweizenmehl und Hartweizengries. Ferner können auch die aufgeführten Dickungs- oder Geliermittel in einer beliebigen Kombination von den aufgeführten Dickungs- oder Geliermitteln aufgeführt werden. Mehrere pflanzliche Dickungs- oder Geliermittel können z.B. in identischen Mengen (Verhältnis 1:1) eingesetzt werden. Die Mengen der mehreren Dickungs- und Geliermittel kann auch unterschiedlich sein. So kann in manchen Ausführungsformen das Verhältnis zweier Geliermittel zwischen 10:1 und 1:10, bevorzugt zwischen 10:1 und 1:5, noch bevorzugter zwischen 5:1 und 1:2 betragen. Beispielhafte Verhältnisse sind 5:1, 4:1, 3:1, 2:1 und 1:2.

In manchen Ausführungsformen umfassen die Hilfsmittel zum Essen oder zum Trinken auch Zellstoff und/oder Holzstoff, was die oben beschriebenen Vorteile mit sich bringt. Weiterhin können die Hilfsmittel zum Essen oder zum Trinken der vorliegenden Erfindung optional Wachs, bevorzugt Carnaubawachs oder Sojawachs, umfassen, was ebenfalls oben beschriebene Vorteile mit sich bringt. Die Hilfsmittel zum Essen oder zum Trinken der vorliegenden Erfindung können optional auch Öl, bevorzugt Nussöl, umfassen.

Ausführungsformen umfassend pflanzliche Stärke und die pflanzliches Dickungs- oder Geliermittel Guarkernmehl und Xanthan

Die folgenden Ausführungsformen beziehen sich auf Hilfsmittel zum Essen oder zum Trinken umfassend pflanzliche Stärke und die pflanzliches Dickungs- oder Geliermittel Guarkernmehl und Xanthan.

Der Gehalt an pflanzlicher Stärke beträgt 40-40%. Ein beispielhafter Stärke-Gehalt ist 50%.

Die pflanzliche Stärke ist Weizenstärke, Kartoffelstärke, Maisstärke, Tapiokastärke oder Stärke aus Maniok, Knollenbohne, Batate, Yamswurzel, Knollen-Platterbse, Arakacha, Knolligem Sauerklee, Knolliger Kapuzinerkresse, Ulluco, Ostindischer Pfeilwurz, Pfeilwurz, Achira, Taro, Tannia, Weißer Seerose, Gelber Teichrose oder Chayote oder eine Mischung davon.

Bevorzugt ist die pflanzliche Stärke Weizenstärke, Kartoffelstärke, Maisstärke, Tapiokastärke oder eine Mischung davon.

Der Gehalt an pflanzlichen Dickungs- und Geliermittel beträgt 35-45. Ein beispielhafter Dickungs- und Geliermittel-Gehalt ist 50%.

Die pflanzliche Dickungs- und Geliermittel sind Guarkernmehl und Xanthan sowie eventuell Agar-Agar, Pektin, Carrageen, Alginat, Johannisbrotkernmehl, Sago, Gummi arabicum, Reismehl, Hartweizenmehl oder Hartweizengries oder eine Mischung davon.

Bevorzugt ist das pflanzliche Dickungs- und Geliermittel eine Mischung aus Guarkernmehl und Xanthan. Die Hilfsmittel zum Essen oder zum Trinken können optional Zellstoff umfassen. Der Gehalt an Zellstoff beträgt 0-10%, bevorzugt 1-10%, noch bevorzugter 2-10%. Ein beispielhafter Zellstoff-Gehalt ist 4%.

Die Hilfsmittel zum Essen oder zum Trinken können optional Holzstoff umfassen. Der Gehalt an Holzstoff beträgt 0-10%, bevorzugt 1-10%, noch bevorzugter 2-10%. Ein beispielhafter Holzstoff-Gehalt ist 4%.

Zellstoff und Holzstoff können in Kombination eingesetzt werden. In diesem Fall beträgt der Gesamt-Gehalt an Zellstoff und Holzstoff 0-10%, bevorzugt 1-10%, noch bevorzugter 2-10%. Ein beispielhafter Gesamt-Gehalt ist 4%. Das Verhältnis von Zellstoff zu Holzstoff beträgt zwischen 10:1 und 1:10, bevorzugt zwischen 10:1 und 1:5, noch bevorzugter zwischen 5:1 und 1:2. Beispielhafte Verhältnisse sind 5:1, 4:1, 3:1, 2:1, 1:1 und 1:2. Der Zellstoff und der Holzstoff weisen bevorzugt Fasern mit einer Länge von 0,8-1,1 mm auf.

Die Hilfsmittel zum Essen oder zum Trinken können optional Wachs umfassen. Der Gehalt an Wachs, bevorzugt Carnaubawachs oder Sojawachs, beträgt 0-10%, bevorzugt 1-10%, noch bevorzugter 1,5-7%. Ein beispielhafter Wachs-Gehalt ist 3%.

Die Hilfsmittel zum Essen oder zum Trinken können optional Öl umfassen. Der Gehalt an Öl, bevorzugt Nussöl, beträgt 0-4%, bevorzugt 0,1-3%. Ein beispielhafter Öl-Gehalt ist 2%.

Die Hilfsmittel zum Essen oder zum Trinken können optional Glycerin umfassen. Der Gehalt an Glycerin 0-10%, bevorzugt 0-3%. Ein beispielhafter Glycerin-Gehalt ist 2%.

In einer weiteren Ausführungsform umfassen die die Hilfsmittel zum Essen oder zum Trinken der vorliegenden Erfindung 40-50% pflanzliche Stärke, 35-45% Guarkernmehl und 1-5% Xanthan.

In einer weiteren Ausführungsform umfassen die die Hilfsmittel zum Essen oder zum Trinken der vorliegenden Erfindung 50% pflanzliche Stärke, 45% Guarkernmehl und 5% Xanthan.

In einer weiteren bevorzugten Ausführungsform umfassen die Hilfsmittel zum Essen oder zum Trinken der vorliegenden Erfindung
40-50% pflanzliche Stärke,
35-45% Guarkernmehl
1-5% Xanthan
0-10% Zellstoff und/oder Holzstoff
0-10% Wachs, bevorzugt Carnaubawachs oder Sojawachs
0-3% Nussöl oder Sonnenblumenöl
0-2% Glycerin.

In einer weiteren bevorzugteren Ausführungsform umfassen die Hilfsmittel zum Essen oder zum Trinken der vorliegenden Erfindung
40-50% pflanzliche Stärke,
35-45% Guarkernmehl
1-5% Xanthan
0-10% Zellstoff und/oder Holzstoff
0,5-10% Wachs, bevorzugt Carnaubawachs oder Rapswachs
0,1-3% Nussöl oder Sonnenblumenöl
0,1-2% Glycerin.

Alle genannten Ausführungsformen können mit einer Wachsschicht versehen werden. Dies wird unter Schritt d des Herstellungsverfahrens erläutert. Die äußere Wachsschicht umfasst Carnaubawachs, Rapswachs, Paraffin, PHA oder Mischungen davon. Die Wachsschicht kann je nach gewünschter Dicke 0-10% der Zusammensetzung des Hilfsmittels betreffen.

In einer weiteren Ausführungsform umfassen die Hilfsmittel zum Essen oder zum Trinken
42% pflanzliche Stärke umfassend Weizenstärke, Maisstärke und/oder Tapiokastärke,
42% Dickungs- oder Geliermittel umfassend Guarkernmehl und Xanthan,
2% Wachs, bevorzugt Carnaubawachs oder Rapswachs,
3% Nussöl oder Sonnenblumenöl,
2% Glycerin und
9% Wachsschicht umfassend Carnaubawachs, Rapswachs, PHA und/oder Paraffin.

Die genannten Ausführungsformen betreffen jegliches Hilfsmittel zum Essen oder zum Trinken. Die Ausführungsformen betreffen demnach Trinkhalme, Besteck, Lollisticks, Sushi Stäbchen, Eisstiele etc.

### Eigenschaften der Hilfsmittel

Die Hilfsmittel zum Essen oder zum Trinken der vorliegenden Erfindung sind biologisch abbaubar und/oder sogar kompostierbar. In manchen Ausführungsformen ist ein Hilfsmittel opak. In anderen Ausführungsformen ist ein Hilfsmittel halb-transparent. Die Farbe der Hilfsmittel zum Essen oder zum Trinken kann mittels herkömmlicher Farbe, bevorzugt Lebensmittelfarbe, modifiziert werden.

**Hilfsmittel zum Trinken** - **Trinkhalm:** Die Wandstärke des Trinkhalms ist variabel. Die Wand des Trinkhalms kann eine Stärke zwischen 0,1 mm und 2 mm, bevorzugt zwischen 0,3 mm und 1,5 mm oder zwischen 0,5 mm und 1,3 mm haben. Beispielhafte Wandstärken sind hier 1 mm und 1,2 mm. Der Durchmesser Trinkhalms ist variabel. Der Durchmesser des Trinkhalms kann zwischen 1 mm und 3 cm, bevorzugt zwischen 3 mm und 1,5 cm oder zwischen 5 mm und 8 mm betragen. Beispielhafte Durchmesser sind hier 6 mm und 7 mm. Die Länge des Trinkhalms ist variabel. Die Länge des Trinkhalms kann zwischen 5 cm und 50 cm, bevorzugt zwischen 10 cm und 35 cm oder zwischen 15 cm und 30 cm betragen. Beispielhafte Längen sind hier 20 cm, 21 cm und 25 cm. In einer Ausführungsform beinhaltet der Trinkhalm zudem Konservierungsstoffe, um ein Verderben des biologisch abbaubaren Materials zu verhindern. Der Gehalt an Konservierungsstoffen beträgt 0-2%, bevorzugt 0,2-1%, noch bevorzugter 0,5%.

In einer Ausführungsform beinhaltet der Trinkhalm zudem Glycerin E 422 (veganes/vegetarisches Glycerin), um die Beweglichkeit bzw. die Biegeeigenschaft der erfindungsgemäßen Trinkhalme zu erhöhen.

Die Trinkhalme der vorliegenden Erfindung sind für eine Vielzahl von Verwendungszwecken geeignet. Sie eignen sich für eine Vielzahl von Getränken, wie z.B. Heißgetränke, Tee, Kaffee, Kaltgetränke, wie Saft, Saftschorlen, Limonaden, Wasser etc. oder alkoholische Getränke, wie z.B. Bier, Wein, Cocktails etc.

**Hilfsmittel zum Essen** - **Besteck:** Die Dicke des Bestecks ist variabel. Die Dicke des Bestecks kann zwischen 1 mm und 20 mm, bevorzugt zwischen 3 mm und 17 mm oder zwischen 5 mm und 15 mm liegen. Eine beispielhafte Dicke ist hier 10 mm.

In einer Ausführungsform beinhaltet das Besteck zudem Konservierungsstoffe, um ein Verderben des biologisch abbaubaren Materials zu verhindern. Der Gehalt an Konservierungsstoffen beträgt 0-2%, bevorzugt 0,2-1%, noch bevorzugter 0,5%.

Das Besteck der vorliegenden Erfindung ist für eine Vielzahl von Verwendungszwecken geeignet. Es eignet sich für eine Vielzahl von Speisen, wie z.B. warme Speisen, Suppen, Eintöpfe, Aufläufe, Fleisch- und Fischgerichte, Beilagen wie beispielsweise Kartoffeln, sowie für kalte Speisen wie Salate, Eis und Joghurt.

**Hilfsmittel zum Essen - Lollistick:** Die Maße eines Lollisticks/Lollistiels der vorliegenden Erfindung sind variabel. Der Durchmesser kann zwischen 1mm und 4mm liegen. Die Wandstärke kann zwischen 0,5 und 1,5mm liegen. In manchen Ausführungen ist der Stick nicht hohl. Die Länge kann zwischen 10 und 15 cm liegen. In einer bevorzugten Ausführung hat ein Lollistick einen Durchmesser von 4mm, eine Wandstärke von 0,5mm und eine Länge von 12 cm. Bei manchen Ausführungen wird in den Endabschnitt des Sticks, der die Zuckermasse trägt, ein Loch in die Wand gestochen. Dies dient zur Halterung der Zuckermasse.

### Herstellungsverfahren

Die vorliegende Erfindung bietet auch Verfahren zur Herstellung von Hilfsmitteln zum Essen oder Trinken gemäß der vorliegenden Erfindung. Dabei umfasst das Verfahren die folgenden Schritte:
a. Herstellen einer Mischung, die pflanzliche Stärke und pflanzliches Dickungs- oder Geliermittel umfasst, umfassend die Schritte
   a.1 Zusammenfügen von festen Bestandteilen der Mischung umfassend pflanzliche Stärke und pflanzliches Dickungs- und Geliermittel und Mischen dieser Bestandteile,
   a.2 Zugabe von flüssigen Bestandteilen umfassend Wasser zur Mischung aus Schritt a.1 in einer Menge von 30-60% des Nettogewichts der Mischung aus a.1 und Mischen dieser Bestandteile,
b. Formen der Mischung aus Schritt a. zu Hilfsmitteln zum Essen oder Trinken, wobei die Mischung aus Schritt a vor dem Formen mittels einer Plastifizierschnecke verdichtet wird,
c. Aushärten des in Schritt b. geformten Hilfsmitteln zum Essen oder Trinken,
d. optional, Beschichten der in Schritt c. ausgehärteten Hilfsmittel zum Essen oder Trinken mit einer Wachsemulsion umfassend Carnaubawachs, Rapswachs, Paraffin, PHA oder Mischungen davon.

### Schritt a:

In Schritt a. werden pflanzliche Stärke und Dickungs- oder Geliermittel mit Wasser vermischt. Dies geschieht mit einem Mixer, der die Mischung zu einer homogenen Masse verrührt. Die Mischung kann optional auch Zellstoff, Holzstoff, Wachs (bevorzugt Carnaubawachs oder Sojawachs) sowie Öl (bevorzugt Nussöl), Glycerin und/oder eine Wachsemulsion umfassen. Hierbei wird folgendermaßen vorgegangen: **In Schritt a.1** werden zunächst die festen Bestandteile umfassend pflanzliche Stärke und Dickungs- und Geliermittel im trockenen, pulverförmigen Zustand gut miteinander vermischt. Dies ist wichtig, um einer Klümpchenbildung beim Mischen mit Wasser entgegenzuwirken.

Die pflanzliche Stärke in der Mischung in Schritt a.1 ist Weizenstärke, Kartoffelstärke, Maisstärke, Tapiokastärke oder Stärke aus Maniok, Knollenbohne, Batate, Yamswurzel, Knollen-Platterbse, Arakacha, Knolligem Sauerklee, Knolliger Kapuzinerkresse, Ulluco, Ostindischer Pfeilwurz, Pfeilwurz, Achira, Taro, Tannia, Weißer Seerose, Gelber Teichrose oder Chayote oder eine Mischung davon.

Bevorzugt ist die pflanzliche Stärke in der Mischung in Schritt a.1 Weizenstärke, Kartoffelstärke, Maisstärke, Tapiokastärke oder eine Mischung davon.

Die pflanzliche Dickungs- und Geliermittel in der Mischung in Schritt a.1 sind Guarkernmehl und Xanthan sowie eventuell Agar-Agar, Pektin, Carrageen, Alginat, Johannisbrotkernmehl, Sago, Gummi arabicum, Reismehl, Hartweizenmehl oder Hartweizengries oder eine Mischung davon.

In manchen Ausführungsformen umfassen die festen Bestandteile in Schritt a1. zudem Zellstoff und/oder Holzstoff. Der Zellstoff kann zumindest teilweise aus Baobab-Pflanzenmaterial oder Bambus-Pflanzenmaterial hergestellt.

Die Mengenanteile der Bestandteile an der Gesamtmenge der Mischung aus Schritt a.1 wird im Folgenden erläutert:
Die Menge an Zellstoff beträgt 0-10%, bevorzugt 1-10%, noch bevorzugter 2-10%. Eine beispielhafte Zellstoff- Menge ist 2%.

Die Menge an Holzstoff beträgt 0-10%, bevorzugt 1-10%, noch bevorzugter 2-10%. Eine beispielhafte Holzstoff- Menge ist 2%.

Zellstoff und Holzstoff können in Kombination eingesetzt werden. In diesem Fall beträgt die Gesamt-Menge an Zellstoff und Holzstoff 0-10%, bevorzugt 1-10%, noch bevorzugter 2-10%. Eine beispielhafte Gesamt-Menge ist 2%. Das Verhältnis von Zellstoff zu Holzstoff beträgt zwischen 10:1 und 1:10, bevorzugt zwischen 10:1 und 1:5, noch bevorzugter zwischen 5:1 und 1:2. Beispielhafte Verhältnisse sind 5:1, 4:1, 3:1, 2:1, 1:1 und 1:2. Der Zellstoff und der Holzstoff weisen bevorzugt Fasern mit einer Länge von 0,8-1,1 mm auf.

In einer Ausführungsform enthält die Mischung in Schritt a.1 der vorliegenden Erfindung
50% pflanzliche Stärke,
45% Guarkernmehl und
5% Xanthan.

Die Menge an pflanzlicher Stärke, Dickungs-oder Geliermittel und Zell-/Holzstoff summiert sich dabei auf eine Gesamtmenge der Mischung aus Schritt a.1 von 100%. Die weiteren Bestandteile der Mischung aus Schritt a (Gesamtmischung) wie zusätzliche feste Bestandteile wie Wachspulver oder die flüssigen Bestandteile aus Schritt a.2, werden am Nettogewicht der Mischung der festen Bestandteile aus Schritt a.1 berechnet und sind daher in % des Nettogewichts der Mischung aus Schritt a.1 angegeben.

**In Schritt a.1 können zusätzliche feste Bestandteileumfasst sein.** In manchen Ausführungsformen umfassen die festen Bestandteile in Schritt a1 zudem Wachspulver, bevorzugt Carnaubawachspulver, Rapswachspulver oder Sojawachspulver.

Die Menge an Wachspulver, bevorzugt Carnaubawachs oder Sojawachs, beträgt 0-10% des Nettogewichts der festen Bestandteile aus der Mischung in Schritt a.1, bevorzugt 1-10%, noch bevorzugter 1,5-4% des Nettogewichts der festen Bestandteile aus der Mischung in Schritt a.1. Eine beispielhafte Wachs-Menge ist 3% des Nettogewichts der festen Bestandteile aus der Mischung in Schritt a.1. Folgendes Beispiel soll die Berechnung für 3% Wachspulver erläutern: Bei einem Nettogewicht der Mischung aus pflanzlicher Stärke, pflanzlichem Dickungs-oder Geliermittel und optional Zell-/Holzstoff von 1000g werden zu dieser Mischung 30g Wachspulver dazugegeben. Danach werden die Bestandteile gut gemischt.

**In** Schritt a.2 werden die flüssigen Bestandteile zur Mischung aus Schritt a.1 in einer Menge bezogen auf das Nettogewicht der Mischung aus a.1 in mL dazu gegeben und gut vermischt. Die flüssigen Bestandteile umfassen Wasser und optional weitere flüssige Bestandteile wie eine Wachsemulsion, Glycerin, und/oder Öl.

Eine bevorzugte Wachsemulsion besteht aus 30-50% Feststoff und 50-70% Wasser, bevorzugt aus 40% Feststoff und 60% Wasser. Diese Emulsion wird unter ständigem Rühren erhitzt bis sich der Feststoff verflüssigt und im Anschluss sofort zur Mischung aus a.1 gegeben. Folgendes Beispiel soll die Berechnung erläutern: Bei einer Zugabe an Wachsemulsion von 2% des Nettogewichts der Mischung aus a.1 beträgt das Volumen der Wachsemulsion bei einem Nettogewicht der Mischung aus a.1 von 1000g 20 mL. Das Wachs umfasst jedes lebensmittelechte Wachs wie Carnaubawachs, Sojawachs, Bienenwachs, Rapswachs, lebensmittelechte Paraffine oder Polyhydroxyalkanoate (PHA) bzw. Polyhydroxyfettsäuren (PHF) oder eine Mischung davon. Bevorzugt wird eine Emulsion aus Carnaubawachs, Sojawachs oder Rapswachs.

Optional wird zur Mischung aus a.1 Öl, bevorzugt Nussöl oder Sonnenblumenöl dazugegeben. Die Menge an zugegebenem Öl beträgt 1-5% des Nettogewichts der Mischung aus a.1 in mL, bevorzugt 2-4% des Nettogewichts der Mischung aus a.1 in mL, noch bevorzugter 4% des Nettogewichts der Mischung aus a.1 in mL. Folgendes Beispiel soll die Berechnung erläutern: Bei einem Nettogewicht der Mischung aus a.1 von 1000g werden zu dieser Mischung 20-40 mL Öl dazugegeben. Danach werden die Bestandteile erneut gut gemischt.

Optional wird zur Mischung aus a.1 Lebensmittelfarbe, bevorzugt in flüssiger Form zugegeben. Der Anteil der Lebensmittelfarbe kann dabei 1-50% des dazugegebenen Wassers ersetzen.

Die Temperatur der Masse beim Mischvorgang liegt zwischen 30 und 40°C, bevorzugt bei 35°C.

Schritt a.2 umfasst beispielhaft:
a.2 Zugabe von flüssigen Bestandteilen der Mischung zur Mischung aus Schritt a.1 umfassend bezogen auf das Nettogewicht der Mischung aus Schritt a.1
2% Carnaubawachsemulsion
4% Sonnenblumenöl
5% Glycerin und
40% Wasser
und Mischen dieser Bestandteile

**Tabelle 1** zeigt weitere Mischungen gemäß Schritt a, d.h. gemäß den Schritten a.1 und a.2.

**Tabelle 1**

| Mischung Schritt a umfassend: | **Feste Bestandteile** (Mischung Schritt a.1) | | | Feste Bestandteile (Angaben in % des Nettogewichts der Mischung der festen Bestandteile aus a.1, in g) | **Flüssige Bestandteile** (Angaben in % des Nettogewichts der Mischung der festen Bestandteile aus a.1, in mL) | | | |
|---|---|---|---|---|---|---|---|---|
| Mischung Schritt a | Pflanzliche Stärke (zB Weizen-, Mais-Kartoffel-, Tapiokastärke) | Pflanzliches Dickungs-und Geliermittel (zB Guarkernmehl, Xanthan, Agar-Agar) | Zellstoff/ Holzstoff | Wachs in Pulverform | Öl (zB Sonnenblumenöl, Nussöl) | Glycerin | Wachsemulsion | Wasser |
| 1 | 50% Maisstärke | 50 % Agar- Agar | | | 4% | 5% | | 40% |
| 2 | 70% | 30% | | | 4% | 10% | | 40% |
| 3 | 60% | 40% | | | 4% | 8% | | 40% |
| 4 | 55% | 45% | | | 4% | 6% | | 40% |
| 5 | 50% | 49% | 1% | 2 % | 3% | 2% | | 40% |
| 6 | 47% | 51% | 2% | 1 % | 2% | 1% | | 40% |
| 7 | 50% | 50% | | 5 % | 0,5% | 15% | | 30% |
| 8 | 50% | 50% | | 8 % | 0,8% | 20% | | 30% |
| 9 | 50% | 50% | 3% | | 1% | 25% | | 30% |
| 10 | 38,5 % | 61,5 % | | 1,5 % | 5% | 5% | | 35% |
| 11 | 52 % | 48% | | | 7% | 4% | | 35% |
| 12 | 33% | 60% | 5% | 2 % | 6% | 3% | | 35% |
| 13 | 30% | 70% | | | 8% | 2% | | 45% |
| 14 | 50% Weizenstärke | 45% Guarkernmehl 5% Xanthan | | | 4% Sonnenblumenöl | 5% | 2% Carnabauwachs | 40% |
| 15 | 55% Maisstärke | 40% Guarkernmehl 5% Xanthan | | 1% Carnabauwachs | 3% Sonnenblumenöl | 5% | | 40% |
| 16 | 52% Maisstärke | 43% Guarkernmehl 5% Xanthan | | | 4% Sonnenblumenöl | 5% | 2% Carnabauwachs | 40% |
| 17 | 50% Tapiokastärke | 45% Guarkernmehl 5% Xanthan | | | 2% Nussöl | | 4% Rapswachs | 30% |
| 18 | 55% Weizenstärke | 40% Guarkernmehl 5% Xanthan | | 2% Sojawachs | 4% Sonnenblumenöl | 10% | | 40% |
| 19 | 65% | 30% Guarkernmehl 5% Xanthan | | | 4% | | | 35% |
| 20 | 50% | 45% Guarkernmehl 5% Xanthan | | | 4% | | | 40% |
| 21 | 50% Weizenstärke | 45% Guarkernmehl 5% Xanthan | | | 4% Sonnenblumenöl | | 2% Carnabauwachs | 40% |
| **Minimum** - **Maximum** | **30%-70%** | **30%-70%** | **0%-10%** | **0%-10%** | **0,5%-10%** | **1%-25%** | **0%-10%** | **30-50%** |

### Schritt b:

Formen der Mischung aus Schritt a zu einem Hilfsmittel zum Essen oder Trinken.

In Schritt b. wird die Mischung aus Schritt a. zu einem Hilfsmittel wie etwa zu einem Trinkhalm geformt. Schritt b wird im Folgenden anhand des Beispiels Trinkhalm erläutert. Durch Änderung der Matrize kann jedoch jegliche Form hergestellt werden.

Die Mischung aus Schritt a wird über ein Auslass-Ventil der Mischvorrichtung in einen Trichter geleitet. Über einen "Screw Conveyor" wird die Mischung weiter transportiert. In einer "Single Screw Extruder"-Maschine, wird die Mischung mittels einer Schnecke (Plastifizierschnecke) zu einem Auslass transportiert und durch einen Auslass in ihre Form gepresst. Dadurch bildet sich die gewünschte Trinkhalm-Form. Die Plastifizierschnecke spitzt sich dabei zum Ende, an welchem sich der Auslass befindet zu, d.h. der Durchmesser der Schnecke wird vom Einlass bis zum Auslass kleiner wodurch der Druck auf die durchlaufende Mischung stets steigt je näher sie dem Auslass kommt. Die Plastifizierschnecke ist selbst in einem Rohr angeordnet welches von verschiedenen Heizbändern ummantelt ist. Zwischen den Heizbändern und dem Rohr ist zudem eine Wasserkühlung angeordnet. Diese gewährleistet keine Überhitzung der Schnecke. Die Heizbänder können unterschiedliche Temperaturen aufweisen. Von Einlass bis Auslass der Schnecke im Rohr sind aufeinanderfolgend Heizbänder angeordnet. Die Temperaturen dieser Heizbänder können 50°C, 65°C, 70°C und 50°C betragen. Durch solch eine Anordnung soll gewährleistet werden, dass die Temperatur des Materials ständig zwischen 50°C und 70°C beträgt. Die Temperatur der Masse bei diesem Schritt ist ausschlaggebend für die Festigkeit und Geschmeidigkeit des späteren Materials. Eine zu niedrige Temperatur bewirkt keine Aktivierung der Gelierungsreaktion was das Material des Trinkhalms spröde werden lässt. Eine zu hohe Temperatur führt jedoch dazu, dass das Material Verbrennungserscheinungen wie Verfärbungen aufweist, spröde und/oder brüchig wird. Eine zu hohe Temperatur kann auch zu Blasenbildung im Material führen. Beides ist unerwünscht.

Die zum Auslass zugespitzte, enger werdende Form der Plastifizierschnecke führt zu immer größer werdender Druckeinwirkung auf das sich durch die Schnecke bewegende Mischung welcher zudem zu einer Temperaturerhöhung führt. Das letzte Heizband ist weniger hoch temperiert, da die Wärme in der Mischung durch Druck und Reibung entsteht. Beste Ergebnisse konnten mit einer Schnecken-Geschwindigkeit von 30 Hz bis 50 Hz erzielt werden. Hierdurch wird bei der Formung der Hilfsmittel das Material der Mischung aus Schritt a sehr dicht zusammengepresst was zu einer hohen Materialdichte führt, die zur Bruchfestigkeit der Trinkhalme beisteuert. Durch diese Materialdichte wird der Trinkhalm sehr hart und bruchsicher. Ferner wird durch den hohen Druck auch das Wasser in der Mischung herausgepresst welches durch Düsen in Form von Wasserdampf abgelassen wird. Das verdichtete Material wird am Auslass der Plastifizierschnecke in ein trichterförmiges Sammelbecken gepresst. Dieses läuft zunächst mit dem Material voll. Im Anschluss wird durch den Druck der aus dem Auslass der Schnecke stets nachfließenden Masse die im Sammelbecken befindliche Masse durch einen weiteren Auslass im Sammelbecken gepresst. Der Auslass umfasst mehrere runde Formen, auch Matrizen genannt, welche zur Formgebung mehrerer Trinkhalme (bzw. anderen länglichen Hilfsmitteln wie Lollistiele oder Haltestiele eines Ohrenstäbchens) führen. Die so durch Extrusion entstehenden (Trinkhalm)Stränge werden auf einen Kamm gelegt und mittels eines Förderbands weiter transportiert. Die geformten Stränge sind heiß (ca. 66°C) und das noch im Material enthaltene Wasser verdampft. Die Stränge für Trinkhalme weisen dabei eine Wandstärke von 1 mm und einen Durchmesser von 7mm auf.

Dieses sogenannte "Pressverfahren" ist sehr zeitsparend, sodass Schritt b in einer Zeitspanne von 4-10 min, bevorzugt 5 min durchgeführt werden kann (von Zugabe der Mischung bis zum gepressten Trinkhalmstrang, welcher den Auslass des Sammelbeckens verlässt).

Dasselbe Verfahren wird zum Formen eines Lollistiels angewandt. Der Durchmesser der Auslässe ist dann geringer. Ein Lollistiel der vorliegenden Erfindung hat einen Durchmesser von 3 mm, eine Wandstärke von 1 mm und eine Länge von 12 cm.

Dasselbe Verfahren wird zum Formen eines Haltestabes für Ohrenstäbchen angewandt.

Formen weiterer Hilfsmittel zum Essen oder Trinken:
Um zum Beispiel Besteck oder andere "flache" Hilfs- oder Bedarfsartikel zu erhalten, wird die Mischung nachdem sie mittels einer Plastifizierschnecke verdichtet worden ist über den Auslass herausgepresst. Der Auslass hat die Form eines nicht geschlossenen Kreises. Sobald die gepresste Masse auf der Oberfläche eines Förderbandes aufliegt, klappen sich die beiden "Kreisenden" zur Seite weg und es entsteht ein flaches Band der Masse. Dieses wird mittels Förderband zu einer Walzmaschine befördert wo es zu Teigplatte mit gewünschter Dicke gewalzt wird. Anschließend werden die gewünschten Hilfsmittel zum Essen oder Trinken wie Bestecke wie Gabel, Löffel und Messer aus der gewalzten Teigplatte mit einem Zylinder ausgestanzt. Die zum Ausstechen verwendete Form erlaubt die Formgebung des jeweilig gewünschten Hilfsmittels durch pressen der flachen Teigplatte in die gewünschte zwei- oder dreidimensionale Form. Dadurch bildet sich die gewünschte Besteck-Form.

### Schritt c:

In Schritt c. werden die in Schritt b geformten Hilfsmittel gehärtet. Dies kann zum Beispiel durch Trocknung mittels Belüftung/Ventilation geschehen. Dabei werden die geformten Hilfsmittel in eine "Cooling und Cutter"-Maschine eingeführt (auch Kühlungstunnel genannt), wo sie durch Ventilatoren getrocknet und dadurch gehärtet werden. Die darauffolgende Härtung mittels Wärme in einem Heiztunnel bzw. Trocknungstunnel wird bei einem Temperaturbereich zwischen 25 und 100°C, bevorzugt bei 85°C durchgeführt. Hierbei wird Luft mit entsprechender Temperatur durch den Tunnel geblasen.

Das Verfahren zur Herstellung des Hilfsmittels kann optional weitere Schritte umfassen. Zum Beispiel, falls das Hilfsmittel ein Trinkhalm, Lollistiel, Haltestiel für Ohrenstäbchen oder ein ähnliches längliches Hilfsmittel ist, können die jeweiligen Stränge, die in Schritt b geformt wurden in der Länge zu einzelnen Trinkhalmen, Lollistielen, Haltestielen für Ohrenstäbchen oder ähnlichen länglichen Hilfsmitteln gekürzt werden. Dies geschieht durch in der "Cooling und Cutter"-Maschine durch beispielsweise ein Messer

Das Verfahren zur Herstellung der Hilfsmittel kann zudem die Bestrahlung der Hilfsmittel mit UV-Licht beinhalten. Dies dient zur Desinfektion der Hilfs- und Bedarfsartikel.

In manchen Ausführungsformen wird das Hilfsmittel zudem bedruckt. Zum Bedrucken können alle dem Fachmann bekannten geeigneten Druckverfahren verwendet werden, z.B. Flexodruckverfahren oder Laserdruckverfahren. Dabei können etwa eine Druckmaschine von Guowei, GWR, oder Hangzhou Colon Machinery Co., Ltd., Modell CL-DC850, zum Einsatz kommen. Der Druck erfolgt bevorzugt mit ungiftigen Farben. In besonders bevorzugten Ausführungsformen wird Lebensmittelfarbe zum Drucken verwendet.

Schritte b und c haben hierbei eine Gesamtdauer von unter 30 min, bevorzugt von 20 min. Das gesamte Herstellungsverfahren weist demnach eine Gesamtdauer von 25-35 min, bevorzugt von 25 min auf. Diese sehr kurze Gesamtdauer schlägt sich in einem geringen Energieverbrauch nieder, was das Verfahren wirtschaftlich macht.

Durch die Verfahren der vorliegenden Erfindung kann eine große Menge an Hilfsmittel zum Essen oder zum Trinken in kurzer Zeit hergestellt werden. Der Output des Verfahrens zur Herstellung von Trinkhalmen kann z.B. bei mindestens 50 kg/h, bevorzugt bei mindestens 100 kg/h, noch bevorzugter bei mindestens 150 kg/h liegen.

Der Output des Verfahrens zur Herstellung von Besteck kann z.B. bei mindestens 50 kg/h, bevorzugt bei mindestens 100 kg/h, noch bevorzugter bei mindestens 150 kg/h liegen.

Zudem ist die hierfür aufzuwendende Energie aufgrund dieser kurzen Zeit im Gegensatz zu üblichen Trocknungsverfahren in z.B. einem Trocknungstunnel sehr gering.

### Schritt d (optional):

Um die Resistenz gegen Feuchtigkeit wie Wasser zu erhöhen, können die durch das vorliegende Verfahren hergestellten Hilfsmittel zum Essen oder Trinken mit einer dünnen Schicht Wachs überzogen werden.

Das Wachs liegt dafür in einer Emulsion vor und wird auf zum Beispiel die Trinkhalme bzw. das Besteck aufgebracht, z.B. aufgesprüht. Bevorzugtes Wachs ist hierbei Carnaubawachs. Eine Bevorzugte Wachemulsion besteht aus 32,5% Carnaubawachs, 17,5% Paraffin und 50% Wasser. Eine weitere bevorzugte Wachsemulsion besteht aus 50% Carnaubawachs und 50% Wasser. Alternativ kann auch Sojawachs, Bienenwachs oder Paraffinwachs verwendet werden aber auch Emulsionen umfassend Polyhydroxyalkanoate (PHA). Die Beschichtung mit der Wachsemulsion kann unterschiedlich erfolgen.

In einer Ausführungsform werden die Hilfs- und Bedarfsartikel kurz in ein Becken mit Wachsemulsion gegeben und danach getrocknet.

In einer Ausführungsform werden die Hilfs- und Bedarfsartikel in eine Beschichtungsmaschine befördert, wo sie mittels Sprühdüsen mit einer Wachsemulsion beschichtet und anschließend getrocknet werden. Eine aufgebrachte Wachsschicht verleiht dem Hilfsmittel zudem eine glatte und glänzende Oberfläche.

### Bevorzugte Ausführungsformen

In einer bevorzugten Ausführungsform umfasst das Verfahren für die Herstellung von Hilfsmitteln zum Essen oder zum Trinken folgende Schritte:
a. Herstellen einer Mischung, die pflanzliche Stärke und pflanzliches Dickungs- oder Geliermittel umfasst, umfassend die Schritte
   a.1 Zusammenfügen der festen Bestandteile der Mischung enthaltend
      50% Weizenstärke
      45% Guarkernmehl
      5% Xanthan
      und Mischen dieser Bestandteile,
   a.2 Zugabe der flüssigen Bestandteile der Mischung zur Mischung aus Schritt a.1 umfassend bezogen auf das Nettogewicht der Mischung aus Schritt a.1
      2% Carnaubawachsemulsion
      4% Sonnenblumenöl
      5% Glycerin und
      40% Wasser
      und Mischen dieser Bestandteile
b. Formen der Mischung aus Schritt a. zu einem Hilfsmittel zum Essen oder Trinken, wobei die Mischung aus Schritt a vor dem Formen mittels einer Plastifizierschnecke verdichtet wird,
c. Aushärten des in Schritt b. geformten Hilfsmittels zum Essen oder Trinken,
d. optional, Beschichten des in Schritt c. ausgehärteten Hilfsmittels zum Essen oder Trinken mit einer Wachsemulsion.

In einer bevorzugten Ausführungsform umfasst das Verfahren für die Herstellung von Hilfsmittel zum Essen oder zum Trinken folgende Schritte:
a. Herstellen einer Mischung, die pflanzliche Stärke und pflanzliches Dickungs- oder Geliermittel umfasst, umfassend die Schritte
   a.1 Zusammenfügen von festen Bestandteilen der Mischung enthaltend
      50% Tapiokastärke
      45% Guarkernmehl
      5% Xanthan
      und Mischen dieser Bestandteile,
   a.2 Zugabe von flüssigen Bestandteilen der Mischung zur Mischung aus Schritt a.1 umfassend bezogen auf das Nettogewicht der Mischung aus Schritt a.1
      4% Rapswachsemulsion
      2% Nussöl
      5% Glycerin und
      30% Wasser
      und Mischen dieser Bestandteile
b. Formen der Mischung aus Schritt a. zu einem Hilfsmittel zum Essen oder Trinken, wobei die Mischung aus Schritt a vor dem Formen mittels einer Plastifizierschnecke verdichtet wird,
c. Aushärten des in Schritt b. geformten Hilfsmittels zum Essen oder Trinken,
d. optional, Beschichten des in Schritt c. ausgehärteten Hilfsmittels zum Essen oder Trinken mit einer Wachsemulsion.

In einer bevorzugten Ausführungsform umfasst das Verfahren für die Herstellung von Hilfsmittel zum Essen oder zum Trinken folgende Schritte:
a. Herstellen einer Mischung, die pflanzliche Stärke und pflanzliches Dickungs- oder Geliermittel umfasst, umfassend die Schritte
   a.1 Zusammenfügen von festen Bestandteilen der Mischung enthaltend
      50% Weizenstärke
      45% Guarkernmehl
      5% Xanthan
      und Mischen dieser Bestandteile,
   a.2 Zugabe von flüssigen Bestandteilen der Mischung zur Mischung aus Schritt a.1 umfassend bezogen auf das Nettogewicht der Mischung aus Schritt a.1
      2% Carnaubawachsemulsion
      4% Sonnenblumenöl
      5% Glycerin und
      40% Wasser
      und Mischen dieser Bestandteile
b. Formen der Mischung aus Schritt a. zu einem Hilfsmittel zum Essen oder Trinken, wobei die Mischung aus Schritt a vor dem Formen mittels einer Plastifizierschnecke verdichtet wird,
c. Aushärten des in Schritt b. geformten Hilfsmittels zum Essen oder Trinken,
d. optional, Beschichten des in Schritt c. ausgehärteten Hilfsmittels zum Essen oder mit einer Wachsemulsion.

### Konkrete Ausführungsformen

Verfahren für die Herstellung von Trinkhalmen umfassend folgende Schritte:
a. Herstellen einer Mischung, die pflanzliche Stärke und pflanzliches Dickungs- oder Geliermittel umfasst, umfassend die Schritte
   a.1 Zusammenfügen von festen Bestandteilen der Mischung enthaltend
      50% Weizenstärke
      45% Guarkernmehl
      5% Xanthan
      und Mischen dieser Bestandteile,
   a.2 Zugabe von flüssigen Bestandteilen der Mischung zur Mischung aus Schritt a.1 umfassend bezogen auf das Nettogewicht der Mischung aus Schritt a.1
      2% Carnaubawachsemulsion
      4% Sonnenblumenöl
      5% Glycerin und
      40% Wasser
      und Mischen dieser Bestandteile
b. Formen der Mischung aus Schritt a. zu einem Trinkhalm, wobei die Mischung aus Schritt a vor dem Formen mittels einer Plastifizierschnecke verdichtet wird,
c. Aushärten des in Schritt b. geformten Trinkhalms,
d. optional, Beschichten des in Schritt c. ausgehärteten Trinkhalms mit einer Wachsemulsion.

Verfahren für die Herstellung von Besteck umfassend folgende Schritte:
a. Herstellen einer Mischung, die pflanzliche Stärke und pflanzliches Dickungs- oder Geliermittel umfasst, umfassend die Schritte
   a.1 Zusammenfügen von festen Bestandteilen der Mischung enthaltend
      50% Weizenstärke
      45% Guarkernmehl
      5% Xanthan
      und Mischen dieser Bestandteile,
   a.2 Zugabe von flüssigen Bestandteilen der Mischung zur Mischung aus Schritt a.1 umfassend bezogen auf das Nettogewicht der Mischung aus Schritt a.1
      2% Carnaubawachsemulsion
      4% Sonnenblumenöl
      5% Glycerin und
      40% Wasser
      und Mischen dieser Bestandteile
b. Formen der Mischung aus Schritt a. zu Besteck, wobei die Mischung aus Schritt a vor dem Formen mittels einer Plastifizierschnecke verdichtet wird,
c. Aushärten des in Schritt b. geformten Bestecks,
d. optional, Beschichten des in Schritt c. ausgehärteten Bestecks mit einer Wachsemulsion.

Verfahren für die Herstellung von Lollisticks umfassend folgende Schritte:
a. Herstellen einer Mischung, die pflanzliche Stärke und pflanzliches Dickungs- oder Geliermittel umfasst, umfassend die Schritte
   a.1 Zusammenfügen von festen Bestandteilen der Mischung enthaltend
      50% Weizenstärke
      45% Guarkernmehl
      5% Xanthan
      und Mischen dieser Bestandteile,
   a.2 Zugabe von flüssigen Bestandteilen der Mischung zur Mischung aus Schritt a.1 umfassend bezogen auf das Nettogewicht der Mischung aus Schritt a.1
      2% Carnaubawachsemulsion
      4% Sonnenblumenöl
      5% Glycerin und
      40% Wasser
      und Mischen dieser Bestandteile
b. Formen der Mischung aus Schritt a. zu einem Lollistick, wobei die Mischung aus Schritt a vor dem Formen mittels einer Plastifizierschnecke verdichtet wird,
c. Aushärten des in Schritt b. geformten Lollisticks,
d. optional, Beschichten des in Schritt c. ausgehärteten Lollisticks mit einer Wachsemulsion.

### Beispiele

Die vorliegende Erfindung wird durch die nachfolgenden, nicht einschränkenden Beispiele, detailliert beschrieben.

### Beispiel 1: Herstellung eines Trinkhalms

Das Ausgangsmaterial für den Trinkhalm bestand aus 50% Weizenstärke, 45% Guarkernmehl, 5% Xanthan Gum. Zusätzlich bezogen auf das Nettogewicht plus 2% Carnaubawachs- Emulsion, 4% Sonnenblumenöl, 5% Glycerin und 40% Wasser. Diese Rohmaterialen, ausschließlich Wasser, Glycerin, CarnaubawachsEmulsion und Sonnenblumen- Öl, wurden in einem Mixer verrührt. Dies geschieht solange bis sich die verschiedenen Bestandteile gut vermischt haben. Daraufhin wurde Wasser (+40% auf das NettoGewicht), Carnaubawachs- Emulsion (+2% auf das Netto- Gewicht), Glycerin (+5% auf das Netto- Gewicht) und Sonnenblumen- Öl (+4% auf das Netto- Gewicht) langsam hinzugegeben. Die sehr pulvrige Zusammensetzung hat eine Temperatur von ca. 35°C. Anschließend wurde diese Mischung über ein Auslass-Ventil in einen Trichter gegeben. Die noch sehr pulvrige Mischung wurde dann in einen "Screw Conveyor", welcher die Mischung weiter nach oben transportierte, gegeben. Anschließend wurde die Mischung in das Fütterungssystem der "Single Screw Extruder"-Maschine befördert. Dieser wurde mittels 4 aufeinander folgenden Heizbändern auf 60°C, 65°C, 65°C, und 50°C erhitzt. Gleichzeitig wurden die Schnecke und der Zylinder durch Wasserkühlung gekühlt. Die Mischung wurde hier durch eine Schnecke nach vorne zu einem Sammelbecken befördert und anschließend durch 10 Auslasse (Matrizen) in ihre Form zu Strängen gepresst. Dadurch bildete sich die gewünschte vorläufige Trinkhalm-Form. Die geformten Trinkhalm-Stränge hatten eine Wandstärke von 1 mm und einen Durchmesser von 7 mm. Die Stränge wurden anschließend in einer "Cooling und Cutter"-Maschine" bearbeitet. Hierbei trockneten die Stränge, bei Durchlaufen des Förderbandes, wo an der Oberseite der Maschine angebrachte Ventilatoren das noch feuchte (35%) und warme (65C°) Material runter kühlten. Nach Durchlaufen des 10m langen Kühlungstunnels, liefen die Stränge in die Schneidemaschine, welche die Stränge in die gewünschte Länge von 20 cm mit einer von oben nach unten stoßenden Klinge zuschnitten. Schließlich wurden die zugeschnittenen Trinkhalme auf das Förderband eines Heiztunnels weiter befördert, welcher die Trinkhalme bei circa 85 °C Heißluft und UV-Licht vollständig aushärtete und desinfizierte. Am Ende des Heiztunnels wurden die Trinkhalme in eine "Beschichtungsmaschine" befördert. Diese befördertet die Trinkhalme mittels eines Förderbandes mit Lamellen in ein Becken mit einer Wachs- Emulsion bestehend aus: 32,5% Carnaubawachs, 17,5% Paraffin und 50% Wasser. Des Weiteren wurden Hilfsstoffe hinzugegeben. Somit wurden die Trinkhalme sowohl innen, als auch außen Beschichtet. Anschließend wurden die Trinkhalme auf einen weiteren Trocknungstunnnel befördert und die Beschichtung härtete weiter aus. Zuletzt fielen die Trinkhalme in einen Karton und wurden anschließend nach Hygienevorschriften verpackt.

### Beispiel 2: Herstellung von Besteck (Messer)

Das Ausgangsmaterial für das Besteck (Messer) besteht aus 55% Maisstärke, 40% Guarkernmehl, 5% Xanthan Gum. Zusätzlich bezogen auf das Nettogewicht plus 4% Rapswachs-Pulver, 4% Nuss-Öl, 35% Wasser und 4% blauer Lebensmittelfarbe. Diese Rohmaterialen, ausschließlich Wasser, Lebensmittelfarbe und Nuss-Öl, wurden in einem Mixer verrührt. Dies geschieht solange bis sich die verschiedenen Bestandteile gut vermischt haben. Daraufhin wurde Wasser (+40% auf das Netto- Gewicht), Lebensmittelfarbe (+4% auf das Netto- Gewicht) und Nuss Öl (+4% auf das Netto- Gewicht) langsam hinzugegeben. Die sehr pulvrige Zusammensetzung hat eine Temperatur von ca. 35°C. Anschließend wurde diese Mischung über ein Auslass-Ventil in einen Trichter gegeben. Die noch pulvrige Mischung wurde dann in einen "Screw Conveyor", welcher die Mischung weiter nach oben transportierte, gegeben. Anschließend wurde die Mischung in das Fütterungssystem des "Single Screw Extruder"-Maschine befördert. Dieser wurde mittels 4 aufeinander folgenden Heizbändern auf 60°C, 65°C, 65°C, und 50°C erhitzt. Gleichzeitig wurden die Schnecke und der Zylinder durch Wasserkühlung gekühlt. Die Mischung wurde hier durch eine Schnecke nach vorne zu einem Sammelbecken befördert und anschließend durch einen Auslass (Matrize) in ihre Form eines fast schließenden Kreises gepresst. Die Masse öffnet sich und faltet sich aus. Dadurch bildete sich die gewünschte vorläufige Form. Mit einer Wandstärke von 2 mm. Die ca. 50 cm breite Teigplatte wird in den Ausstanz-Bereich befördert. Hierbei wird die Masse mit einer Walzmaschine zu der gewünschten Form (Messer) aus der flachen (2 mm) Teigplatte ausgestochen. Dadurch bildete sich die gewünschte Besteck-Form, in diesem Fall ein Messer. Das geformte Messer weist eine Dicke von 2 mm auf. Schließlich wurde das zugeschnittene Besteck (Messer) auf das Förderband eines Heiztunnels befördert, welcher das Besteck (Messer) bei circa 85°C Heißluft und UV-Licht vollständig aushärtete und desinfizierte. Am Ende des Heiztunnels wurden die Messer in eine Beschichtungsmaschine befördert. Diese beschichtete die Messer mittels Sprühdüsen mit einer Wachs- Emulsion aus 50% Carnaubawachs, und 50% Wasser. Des Weiteren wurden Hilfsstoffe hinzugegeben. Das Messer drehte sich hierbei einmal von einer Seite zur anderen, durch die sich selbst drehenden Zylinder, die gleichzeitig durch das Drehen die Messer nach vorne beförderte und durch darüber angebrachte Ventilatoren trockneten. Zuletzt fielen die Messer in einen Karton und wurden anschließend nach Hygienevorschriften verpackt.

### Beispiel 3: Herstellung Eisstiel

Das Ausgangsmaterial für den Eis-Stiel besteht aus 50% Tapiokastärke, 45% Guarkernmehl, 5% Xanthan Gum. Zusätzlich bezogen auf das Nettogewicht plus 2% Nussöl, 4% Rapswachs- Emulsion und 30% Wasser. Diese Rohmaterialen, ausschließlich Wasser, Rapswachs- Emulsion und Öl, wurden in einem Mixer verrührt. Dies geschieht solange bis sich die verschiedenen Bestandteile gut vermischt haben. Daraufhin wurde Wasser (+40% auf das Netto- Gewicht), Rapswachs- Emulsion (+4% auf das Netto- Gewicht) und Nuß- Öl (+2% auf das Netto- Gewicht) langsam hinzugegeben. Die sehr pulvrige Zusammensetzung hat eine Temperatur von ca. 35°C. Anschließend wurde diese Mischung über ein Auslass-Ventil in einen Trichter gegeben. Die noch pulvrige Mischung wurde dann in einen "Screw Conveyor", welcher die Mischung weiter nach oben transportierte, gegeben. Anschließend wurde die Mischung in das Fütterungssystem des "Single Screw Extruder"-Maschine befördert. Dieser wurde mittels 4 aufeinander folgenden Heizbändern auf 60°C, 65°C, 65°C, und 50°C erhitzt. Gleichzeitig wurden die Schnecke und der Zylinder durch Wasserkühlung gekühlt. Die Mischung wurde hier durch eine Schnecke nach vorne zu einem Sammelbecken befördert und anschließend durch einen Auslass (Matrize) in ihre Form eines fast schließenden Kreises gepresst. Die Masse öffnet sich und faltet sich aus. Dadurch bildete sich die gewünschte vorläufige Form. Mit einer Wandstärke von 2 mm. Die 50 cm breite Teigplatte wird in den Ausstanz- Bereich befördert. Hierbei wird die Masse mit einer Walzmaschine zu dem gewünschten der Form, Eis-Stiel aus der flachen (2 mm) Teigplatte, ausgestochen. Dadurch bildete sich die gewünschte Eisstiel- Form. Der geformte Eisstiel weist eine Dicke von 2 mm auf. Schließlich wurde der zugeschnittene Eisstiel auf das Förderband eines Heiztunnels befördert, welcher den Eisstiel bei circa 80°C Heißluft und UV-Licht vollständig aushärtete und desinfizierte. Am Ende des Heiztunnels wurden die Eisstiele in eine Beschichtungsmaschine befördert. Diese beschichtete die Eisstiele mittels Sprühdüsen mit einer WachsEmulsion aus (50% Sojawachs, und 50% Wasser) Des Weiteren wurden Hilfsstoffe hinzugegeben. Der Eisstiel drehte sich hierbei einmal von einer Seite zur anderen, durch die sich selbst drehenden Zylinder, die gleichzeitig durch das Drehen die Eisstiele nach vorne beförderte und durch darüber angebrachte Ventilatoren trockneten. Zuletzt fielen die Eisstiele in einen Karton und wurden anschließend nach Hygienevorschriften verpackt.

## Patentansprüche

1. Hilfsmittel zum Essen oder zum Trinken umfassend
40-50% pflanzliche Stärke,
35-45% Guarkernmehl und
1-5% Xanthan.

2. Hilfsmittel zum Essen oder zum Trinken gemäß Anspruch 1, ferner umfassend eine äußere Wachsschicht umfassend Carnaubawachs, Rapswachs, Paraffin, PHA oder Mischungen davon.

3. Hilfsmittel zum Essen oder zum Trinken gemäß Anspruch 1 oder 2, wobei die pflanzliche Stärke Weizenstärke, Kartoffelstärke, Maisstärke, Tapiokastärke oder eine Mischung davon ist und wobei das pflanzliche Dickungs- oder Geliermittel eine Mischung aus Guarkernmehl und Xanthan ist.

4. Hilfsmittel zum Essen oder zum Trinken gemäß einem der Ansprüche 1-3, wobei das Hilfsmittel ein Trinkhalm, ein Besteck oder ein Lollistiel ist.

5. Hilfsmittel zum Essen oder zum Trinken gemäß einem der Ansprüche 1-4, wobei das Hilfsmittel zum Essen oder zum Trinken kompostierbar und/oder vollständig biologisch abbaubar ist.

6. Verfahren zur Herstellung von Hilfsmitteln zum Essen oder Trinken nach einem der Ansprüche 1-5, wobei das Verfahren die folgenden Schritte umfasst:
a. Herstellen einer Mischung, die pflanzliche Stärke, Guarkernmehl und Xanthan umfasst, umfassend die Schritte
a.1 Zusammenfügen von festen Bestandteilen der Mischung umfassend pflanzliche Stärke, Guarkernmehl und Xanthan und Mischen dieser Bestandteile,
a.2 Zugabe von flüssigen Bestandteilen umfassend Wasser zur Mischung aus Schritt a.1 in einer Menge von 30-60% des Nettogewichts der Mischung aus a.1 und Mischen dieser Bestandteile,
b. Formen der Mischung aus Schritt a. zu einem Hilfsmittel zum Essen oder Trinken, wobei die Mischung aus Schritt a vor dem Formen mittels einer Plastifizierschnecke verdichtet wird,
c. Aushärten des in Schritt b. geformten Hilfsmittels zum Essen oder Trinken,
d. optional, Beschichten des in Schritt c. ausgehärteten Hilfsmittels zum Essen oder Trinken mit einer Wachsemulsion umfassend Carnaubawachs, Rapswachs, Paraffin, PHA oder Mischungen davon.

7. Verfahren zur Herstellung von Hilfsmitteln zum Essen oder Trinken nach Anspruch 6, wobei die flüssigen Bestandteile aus Schritt a.2 ferner Öl, bevorzugt Nussöl oder Sonnenblumenöl, Glycerin und/oder eine Wachsemulsion umfassen.

8. Verfahren zur Herstellung von Hilfsmitteln zum Essen oder Trinken nach Anspruch 6 oder 7, wobei das Verfahren die folgenden Schritte umfasst:
a. Herstellen einer Mischung, die pflanzliche Stärke und pflanzliches Dickungs- oder Geliermittel umfasst, umfassend die Schritte
a.1 Zusammenfügen von festen Bestandteilen der Mischung enthaltend
50% Weizenstärke
45% Guarkernmehl
5% Xanthan
und Mischen dieser Bestandteile,
a.2 Zugabe von flüssigen Bestandteilen der Mischung zur Mischung aus Schritt a.1 umfassend
bezogen auf das Nettogewicht der Mischung aus Schritt a.1
2% Carnaubawachsemulsion
4% Sonnenblumenöl
5% Glycerin und
40% Wasser
und Mischen dieser Bestandteile
b. Formen der Mischung aus Schritt a. zu einem Hilfsmittel zum Essen oder Trinken,
c. Aushärten des in Schritt b. geformten Hilfsmittels zum Essen oder Trinken,
d. optional, Beschichten des in Schritt c. ausgehärteten Hilfsmittels zum Essen oder Trinken mit einer Wachsemulsion.

9. Verfahren zur Herstellung von Hilfsmitteln zum Essen oder Trinken nach einem der Ansprüche 6-8, wobei die Wachsemulsion im optionalen Schritt d. entweder aus 32,5% Carnaubawachs, 17,5% Paraffin und 50% Wasser oder aus 50% Carnaubawachs und 50% Wasser besteht.

## Claims

1. Aids for eating or drinking comprising
40-50% vegetable starch and
35-45% guar gum and
1-5% xanthan gum.

2. Aid for eating or drinking according to claim 1, further comprising an outer wax coating comprising carnauba wax, rapeseed wax, paraffin, PHA or mixtures thereof.

3. Aid for eating or drinking according to claim 1 or 2, wherein the vegetable starch is wheat starch, potato starch, corn starch, tapioca starch or a mixture thereof and wherein the vegetable thickening or gelling agent is a mixture of guar gum and xanthan gum.

4. Aid for eating or drinking according to any one of claims 1-3, wherein the aid is a drinking straw, a cutlery or a lollipop stick.

5. Aid for eating or drinking according to any one of claims 1-4, wherein the aid for eating or drinking is compostable and/or fully biodegradable.

6. A method of making aids for eating or drinking according to any one of claims 1-5, the method comprising the steps of
a. preparing a mixture comprising vegetable starch, guar gum and xanthan gum comprising the steps of
a.1 combining solid ingredients of the mixture comprising vegetable starch guar gum and xanthan gum and mixing these ingredients,
a.2 adding liquid ingredients comprising water in an amount of 30-60% of the net weight of the mixture of a.1 to the mixture of step a.1 and mixing these ingredients,
b. forming the mixture of step a. into an aid for eating or drinking, wherein the mixture of step a. is densified by means of a plasticizing screw prior to forming,
c. curing the aid for eating or drinking formed in step b.,
d. optionally, coating the aid for eating or drinking cured in step c. with a wax emulsion comprising carnauba wax, rapeseed wax, paraffin, PHA or mixtures thereof.

7. The method of making aids for eating or drinking claim 6, wherein the liquid ingredients of step a.2 further comprise oil, preferably nut oil or sunflower oil, glycerol and/or a wax emulsion.

8. The method of making aids for eating or drinking according to claim 6 or 7, the method comprising the steps of
a. preparing a mixture comprising vegetable starch and vegetable thickening or gelling agent comprising the steps of
a.1 combining solid ingredients of the mixture including
50% wheat starch
45% guar gum
5% xanthan gum
and mixing these ingredients,
a.2 adding liquid ingredients to the mixture of step a.1 comprising
2% carnauba wax
4% sunfloweroil
5% glycerol
40% water
of the net weight of the mixture of step a.1
and mixing these ingredients,
b. forming the mixture of step a. into an aid for eating or drinking,
c. curing the aid for eating or drinking formed in step b.,
d. optionally, coating the aid for eating or drinking cured in step c. with a wax emulsion.

9. The method of making aids for eating or drinking according to any of claims 6-8, wherein the wax emulsion of optional step d. consists either of 32.5% carnauba wax, 17.5% paraffin and 50% water, or of 50% carnauba wax and 50% water.

## Revendications

1. Auxiliaire pour manger ou pour boire comprenant
40-50 % d'amidon végétal,
35-45 % de gomme de guar et
1-5 % de xanthane.

2. Auxiliaire pour manger ou pour boire selon la revendication 1, comprenant en outre une couche de cire extérieure comprenant de la cire de carnauba, de la cire de colza, de la paraffine, du PHA (polyhydroxyalcanoate) ou des mélanges de ceux-ci.

3. Auxiliaire pour manger ou pour boire selon la revendication 1 ou 2, dans lequel l'amidon végétal est de l'amidon de blé, de l'amidon de pomme de terre, de l'amidon de maïs, de l'amidon de tapioca ou un mélange de ceux-ci, et dans lequel l'agent épaississant ou gélifiant végétal est un mélange composé de gomme de guar et de xanthane.

4. Auxiliaire pour manger ou pour boire selon l'une quelconque des revendications 1 - 3, dans lequel l'auxiliaire est une paille, un couvert ou un bâtonnet de sucette.

5. Auxiliaire pour manger ou pour boire selon l'une quelconque des revendications 1 - 4, dans lequel l'auxiliaire pour manger ou pour boire est compostable et/ou totalement biodégradable biologiquement.

6. Procédé de fabrication d'auxiliaires pour manger ou boire selon l'une quelconque des revendications 1 - 5, dans lequel le procédé comprend les étapes suivantes :
a. la fabrication d'un mélange qui comprend de l'amidon végétal, de la gomme de guar et du xanthane, comprenant les étapes
a.1 d'assemblage de composants solides du mélange comprenant de l'amidon végétal, de la gomme de guar et du xanthane et de mélange desdits composants,
a.2 d'ajout de composants liquides comprenant de l'eau au mélange issu de l'étape a.1 en une quantité de 30-60 % du poids net du mélange issu de a.1 et de mélange desdits composants,
b. le façonnage du mélange issu de l'étape a. en un auxiliaire pour manger ou boire, dans lequel le mélange issu de l'étape a est compacté avant le façonnage au moyen d'une vis sans fin de plastification,
c. le durcissement de l'auxiliaire pour manger ou boire façonné lors de l'étape b.,
d. en option le revêtement de l'auxiliaire pour manger ou boire durci lors de l'étape c. d'une émulsion de cire comprenant de la cire de carnauba, de la cire de colza, de la paraffine, du PHA ou des mélanges de ceux-ci.

7. Procédé de fabrication d'auxiliaires pour manger ou boire selon la revendication 6, dans lequel les composants liquides issus de l'étape a.2 comprennent en outre de l'huile, de manière préférée de l'huile de noix ou de l'huile de tournesol, de la glycérine et/ou une émulsion de cire.

8. Procédé de fabrication d'auxiliaires pour manger ou boire selon la revendication 6 ou 7, dans lequel le procédé comprend les étapes suivantes :
a. la fabrication d'un mélange qui comprend de l'amidon végétal et un agent épaississant ou gélifiant végétal, comprenant les étapes
a.1 d'assemblage de composants solides du mélange contenant
50 % d'amidon de blé,
45 % de gomme de guar
5 % de xanthane
et de mélange desdits composants,
a.2 d'ajout de composants liquides du mélange au mélange issu de l'étape a.1 comprenant
par rapport au poids net du mélange issu de l'étape a.1
2 % d'émulsion de cire de carnauba
4 % d'huile de tournesol
5 % de glycérine et
40 % d'eau
et de mélange desdits composants
b. le façonnage du mélange issu de l'étape a. en un auxiliaire pour manger ou boire,
c. le durcissement de l'auxiliaire pour manger ou boire façonné lors de l'étape b.
d. en option, le revêtement de l'auxiliaire pour manger ou boire durci lors de l'étape c. d'une émulsion de cire.

9. Procédé de fabrication d'auxiliaires pour manger ou boire selon l'une quelconque des revendications 6 - 8, dans lequel l'émulsion de cire est constituée dans l'étape d. optionnelle soit de 32,5 % de cire de carnauba, 17,5 % de paraffine et 50 % d'eau soit de 50 % de cire de carnauba et 50 % d'eau.
